## Europäisches Patentamt
## European Patent Office
## Office européen des brevets

(19)

(11) Numéro de publication: **0 107 580**
**B1**

(12)
# FASCICULE DE BREVET EUROPÉEN

(45) Date de publication du fascicule du brevet:
**08.04.87**

(21) Numéro de dépôt: **83402000.0**

(22) Date de dépôt: **13.10.83**

(51) Int. Cl.⁴: **G 01 N 21/82,** G 01 N 21/25, G 01 N 33/80

(54) **Dispositif pour la détection et la quantification d'agglutinats.**

(30) Priorité: **21.10.82 FR 8217635**

(43) Date de publication de la demande:
**02.05.84 Bulletin 84/18**

(45) Mention de la délivrance du brevet:
**08.04.87 Bulletin 87/15**

(84) Etats contractants désignés:
**AT BE CH DE GB IT LI LU NL SE**

(56) Documents cité:
**DE-A-2 553 129**
**DE-A-3 306 491**
**FR-A-2 475 735**
**US-A-3 883 308**

(73) Titulaire: **LE MATERIEL BIOMEDICAL Société à Responsabilité Limitée dite:, 4 rue de Presbourg, F-75116 Paris (FR)**

(72) Inventeur: **Benajam, Alain Charles Albert, 36 avenue Karl Marx, F-93000 Bobigny (FR)**

(74) Mandataire: **Bonnetat, Christian, Cabinet PROPI Conseils 23 rue de Léningrad, F-75008 Paris (FR)**

## Description

La présente invention concerne un dispositif pour la détection et la quantification d'agglutinats susceptibles d'être formés, sous l'action d'au moins un réactif, par des particules en suspension dans un liquide.

Elle peut être utilisée chaque fois que l'on a à détecter et à quantifier des agglutinats. Cependant, elle s'applique particulièrement bien en immunohématologie, notamment en vue de la détermination des groupes sanguins. On sait en effet que la détermination des groupes sanguins est liée à la recherche de l'existence dans le sang d'antigènes érythrocytaires. Les réactions immunocytologiques, face à un antigène de groupe spécifique, se traduisent par un phénomène d'agglutination des hématies, si ces dernières appartiennent à un autre groupe. Ces hématies peuvent former soit des agglutinats uniformes sans hématies libres, soit quelques gros agglutinats, ou bien encore un grand nombre de petits agglutinats.

Quoique la présente invention ne soit pas limitée dans ses applications à l'immunohématologie, elle sera décrite ci-après plus particulièrement en rapport avec cette application particulière.

Selon l'invention, le dispositif pour la détection et la quantification, dans des récipients dont au moins les fonds sont transparents, d'agglutinats susceptibles d'être formés, sous l'action d'au moins un réactif liquide, par des particules en suspension dans des doses de liquides à tester se trouvant dans lesdits récipients, dispositif comportant un agencement linéaire d'une pluralité n d'éléments photosensibles observant par transparence les fonds desdits récipients contenant les éventuels agglutinats en balayant lesdits fonds desdits récipients, de façon à former n x m points d'observation répartis selon une surface rectangulaire ou carrée occupant la plus grande partie de la surface du fond de chaque récipient, lesdits récipients étant constitués d'alvéoles répartis selon des rayons d'un disque horizontal transparent pouvant tourner autour de son axe vertical, est remarquable en ce qu'un bras oscillant tournant autour d'un axe vertical et pourvu de moyens de prélèvement et de restitution de doses liquides est prévu pour prélever de telles doses dans des réservoirs à réactifs et à liquides à tester et les amener dans lesdits alvéoles du disque tournant, en ce que ledit agencement linéaire d'éléments photosensibles est disposé à poste fixe orthogonalement à un diamètre du disque transparent et en ce que l'axe de rotation du disque est monté mobile en translation orthogonalement audit agencement linéaire d'éléments photosensibles.

Ainsi, par l'intermédiaire de ses moyens de prélèvement et de restitution de doses liquides, qui peuvent par exemple être des seringues, le bras oscillant peut introduire dans chaque alvéole du disque tournant un liquide à tester et le réactif correspondant. Après réaction, chaque alvéole est examiné par l'agencement d'éléments photosensibles au cours de la translation de l'axe du disque, translation dont l'amplitude est suffisante pour que tous les alvéoles d'un rayon du disque soit observé, chacun en m pas.

Avantageusement, le bras oscillant porte autant de moyens de prélèvement et de restitution qu'un rayon de disque comporte d'alvéoles de sorte que le bras oscillant charge tous les alvéoles d'un rayon en une seule fois.

Dans un mode avantageux de réalisation, l'agencement linéaire d'éléments photosensibles est disposé orthogonalement au plan vertical passant par les axes verticaux du disque transparent et du bras oscillant, du côté de l'axe du disque opposé audit bras oscillant, de sorte que le disque doit effectuer une rotation de 180° pour qu'un rayon d'alvéoles chargés en doses de liquides à tester et en réactif puisse être lu par ledit agencement.

De préférence, les réservoirs à réactifs sont disposés à poste fixe d'un côté du plan vertical défini par les axes verticaux de bras oscillant et du disque, tandis que les réservoirs à liquides à tester sont associés à des moyens de transport les avançant pas à pas, parallèlement à ce plan vertical, mais du côté opposé auxdits réservoirs de réactifs.

L'agencement d'éléments photosensibles peut être du type barrette de diodes à couplage de charges, désignées généralement par les lettres CCD, DTC ou CCPD. Selon l'invention, dans laquelle on utilise une barrette rectiligne de n dispositifs à couplage de charges, on observe, en vue de la détection des éventuels agglutinats et lors du mouvement relatif de chaque fond transparent du récipient suivant m positions écartées et parallèles de ladite barrette, de façon à former n x m points d'observation répartis selon une surface rectangulaire ou carrée occupant la plus grande partie de la surface dudit fond. Bien entendu, on peut choisir n égal à m et faire en sorte que l'écart entre deux positions d'observation consécutives de la barrette soit égal à l'écart entre deux dispositifs CCD de la barrette. Ainsi, on couvre totalement une surface carrée du fond transparent. Par exemple, la barrette rectiligne comporte 256 photodiodes CCD, ayant chacune une définition de 25 μm, l'information vidéo étant numérisée en 256 niveaux de gris, et l'image analysée représente un carré centré sur ledit fond transparent et formé par 256 balayages écartés de 25 μm.

On obtient ainsi, pour l'observation des agglutinats et pour chaque récipient, n x m mesures, réparties en m balayages de n points.

Les figures du dessin annexé feront bien comprendre comment l'invention peut être réalisée.

La figure 1 est une vue d'ensemble en perspective, avec arrachement partiel, d'une installation avec dispositif selon l'invention.

La figure 2 est une vue de dessus simplifiée du dispositif selon l'invention.

La figure 3 est une vue de dessus d'un disque à alvéoles utilisé dans le dispositif selon l'invention.

La figure 4 est une coupe selon la ligne IV-IV de la figure 3.

La figure 5 montre en perspective un porte-échantillons pour le dispositif selon l'invention.

Les figures 6 et 7 sont des coupes respectivement selon les lignes VI-VI et VII-VII de la figure 2.

La figure 8 illustre schématiquement le processus de lecture des agglutinats.

Sur ces figures des références identiques designent des éléments identiques.

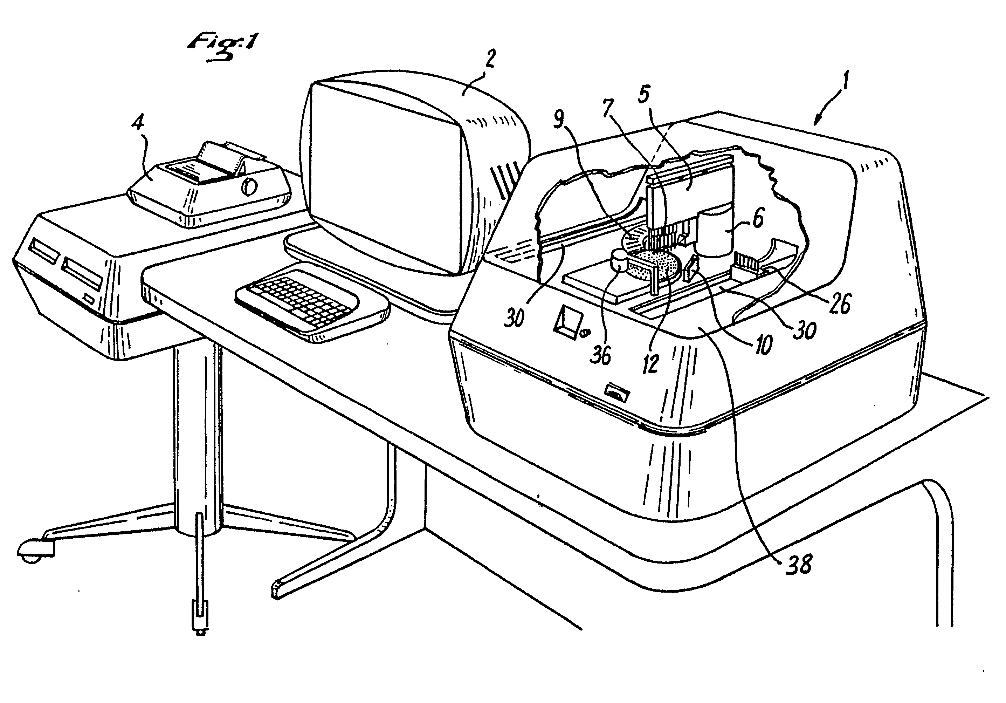

L'installation pour la reconnaissance des groupes sanguins, montrée par la figure 1, comporte un dispositif 1 selon l'invention pour la détection et la quantification d'agglutinats, associé à un dispositif de visualisation 2, à un dispositif de commande 3 et à un dispositif d'impression 4. L'ensemble de l'installation est pilotée par un microprocesseur (non représenté) selon un processus non décrit ci-après.

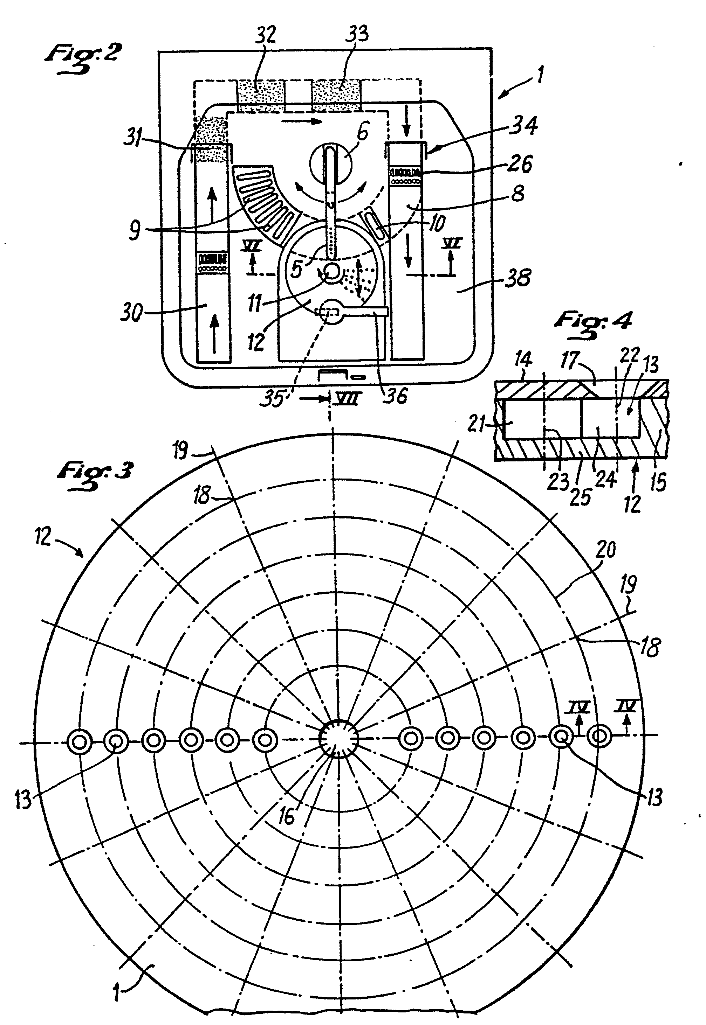

Comme le montre également la figure 2, le dispositif 1 selon l'invention comporte un bras oscillant horizontal 5 susceptible de tourner autour d'un axe vertical 6. Le bras 5 porte une pluralité de seringues verticales 7, dont les pistons peuvent être actionnés pour aspirer ou refouler un liquide. Lorsque le bras 5 oscille autour de l'axe vertical 6, les seringues 7 balayent une zone annulaire 8.

Dans cette zone annulaire 8 sont prévus des récipients à réactifs 9 dans chacun desquels les seringues verticales 7 peuvent prélever un réactif et un récipient de rinçage 10.

Par ailleurs, le dispositif 1 comporte un axe vertical 11 susceptible d'entraîner en rotation un disque 12 de matière transparente dans lequel sont pratiqués des alvéoles 13. Par exemple, comme le montrent les figures 3 et 4, le disque 12 comporte un disque supérieur 14 et un disque inférieur 15 superposés et assemblés par exemple par collage. Les disques 14 et 15 sont en une matière synthétique transparente et sont pourvus en leur centre de trous 16 pour permettre le passage de l'arbre rotatif d'entraînement 11 lorsque les disques 1 et 2 sont superposés et solidarisés.

Le disque supérieur 14 est pourvu d'une pluralité de trous traversants 17 dont les axes 22 orthogonaux au plan du disque 14 sont répartis en une pluralité de points 18 se trouvant à l'intersection de rayons 19 et de cercles concentriques 20. Par ailleurs, chaque trou 17 est évasé vers le haut.

A des fins de clarté du dessin, sur la figure 3, seuls quelques trous 17 ont été représentés.

Le disque transparent inférieur 15 comporte une pluralité de trous borgnes 21 dont les axes 23 sont répartis en une pluralité de points se trouvant à l'intersection de rayons et de cercles concentriques et sont décalés par rapport aux axes 22. Les trous borgnes 21 sont, dans le disque inférieur 15, prolongés en direction des trous 17, par un trou de communication 24 à fond plat, suffisamment allongé pour que le bord supérieur des entonnoirs 17 se trouve à l'extérieur du récipient 21, ou tout au moins dégage sur le fond de celui-ci un champ de vision 25 recouvrant pratiquement la plus grande partie dudit fond.

La zone annulaire 8 de balayage des seringues 7 coupe le disque 12 de sorte qu'il est possible de remplir tous les alvéoles 13 (ou récipient 13) disposés sur un rayon 19 du disque 12, en doses de réactifs provenant des réservoirs 9 en introduisant les aiguilles de seringues 7 dans les entonnoirs correspondants 17, le liquide traversant alors les trous 17 et 24 pour parvenir jusqu'au trou borgne 21. De la même façon, il est possible d'introduire dans les récipients 13 des doses de liquides provenant de réservoirs 26 se trouvant dans la zone 8. Lorsqu'une agitation ou une centrifugation est nécessaire, il suffit de soumettre le disque 12 à ce mouvement et, puisque les trous 21 sont obturés par le disque 14 il n'y a aucun risque que le liquide contenu dans les récipients 13 s'échappe vers l'extérieur.

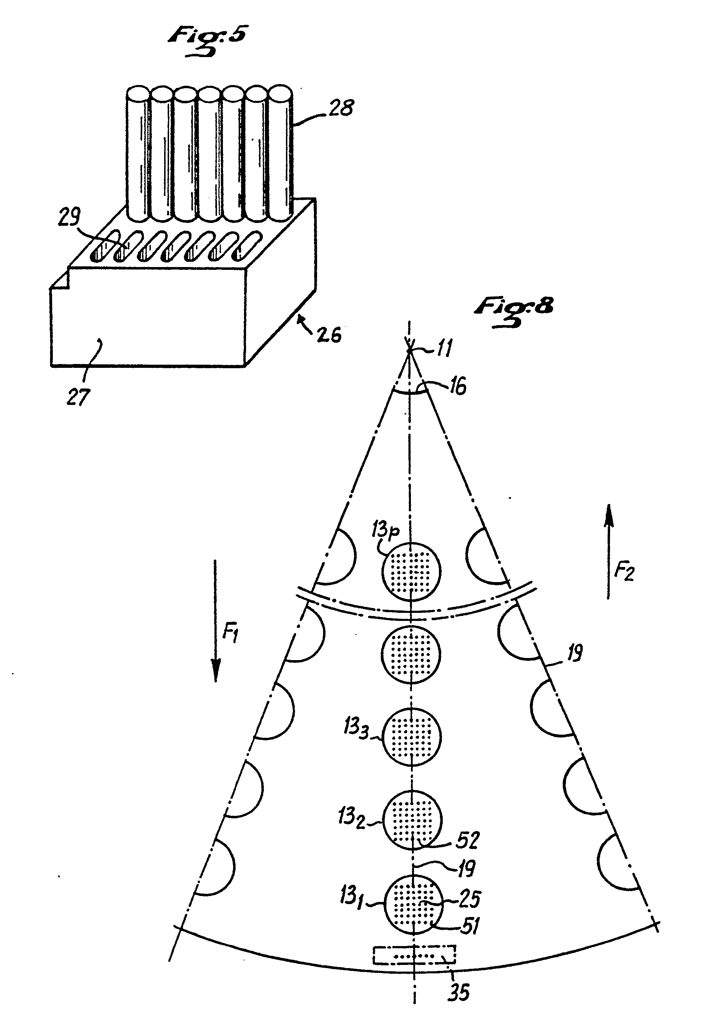

Les réservoirs 26 sont constitués (voir la figure 5) de blocs transparents 27 portant de façon amovible des tubes 28 qui contiennent des échantillons de sang provenant de donneurs et dans lesquels sont éventuellement pratiqués des évidements 29 contenant des dilutions des liquides desdits tubes.

Les réservoirs 26 sont introduits dans le dispositif 1 par un convoyeur 30 qui les fait passer successivement à différents postes de traitement 31, 32 et 33, avant de les amener au poste 34 où les seringues 7 du bras 5 peuvent prélever le liquide qu'ils contiennent.

Ainsi, chaque alvéole 13 du disque 12 peut servir de récipient de réaction entre un liquide provenant d'un réservoir 26 et un réactif provenant d'un réservoir 9.

Pour examiner le résultat de l'agglutination qui peut en résulter, le dispositif 1 comporte une barrette 35 de diodes CCD, disposée sous le disque 12 en regard d'un dispositif d'éclairage 36. La barrette 35 et le dispositif d'éclairage 36 sont opposés au bras 5 par rapport à l'axe 11 et la barrette 35 est orthogonale au plan vertical défini par les axes 6 et 11.

La barrette 35 examine par transparence le fond 25 de chaque alvéole 13 selon m observations décalées chacune d'un pas. Pour que tous les alvéoles 13 d'un rayon 19 du disque 12 puissent être examinés chacun en m pas, l'axe 11 dudit disque est rendu mobile en translation horizontale, orthogonalement à ladite barrette 35.

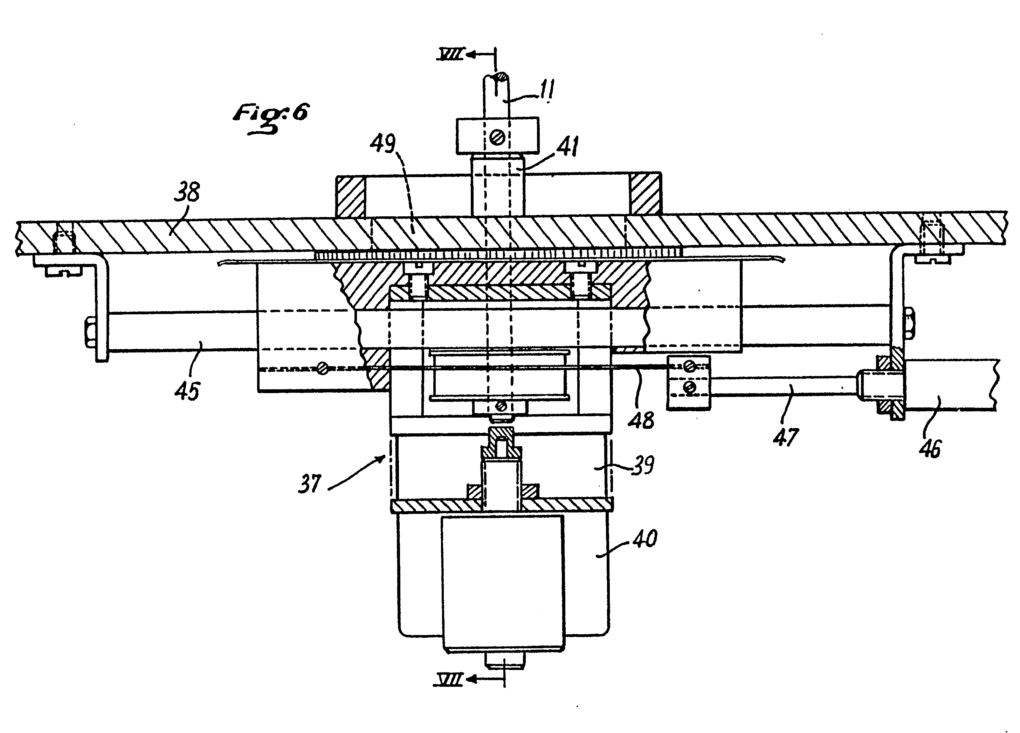

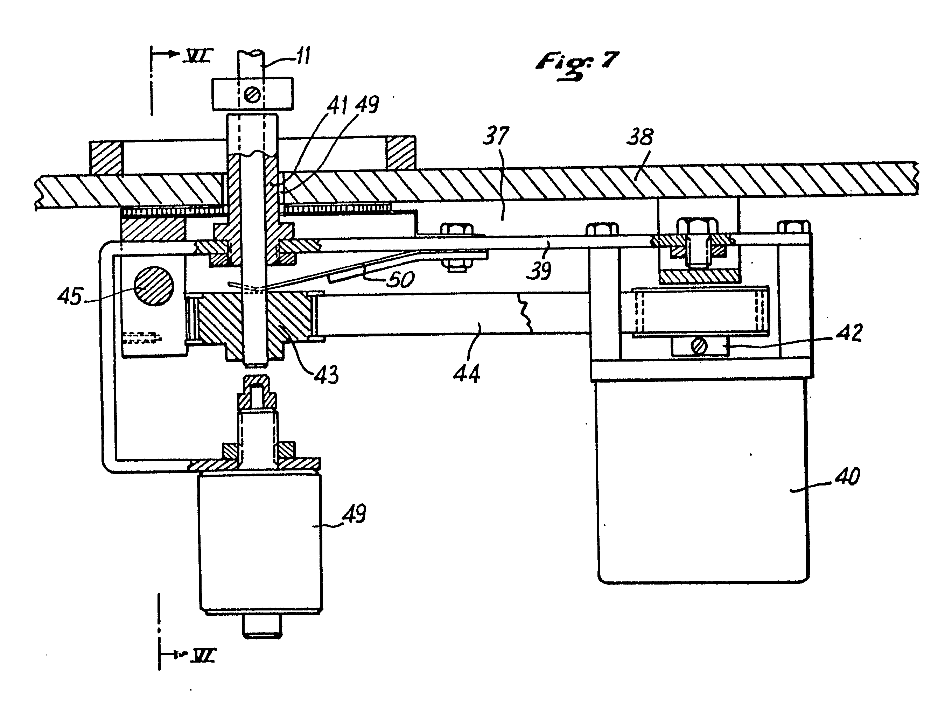

A cette fin, les figures 6 et 7 montrent que l'axe 11 du disque 12 est solidaire d'un chariot 37 mobile par rapport à la plaque de base 38 du dispositif. Ce chariot 37 comporte un bâti 39 portant un moteur 40 et un palier lisse 41 pour l'axe 11. L'arbre de sortie du moteur 40 entraîne en rotation une poulie 42 qui est reliée à une poulie 43 calée sur l'axe 11, par l'intermédiaire d'une courroie crantée 44. Le chariot 37 est guidé en translation par un rail 45 et il se déplace sous l'action d'un vérin 46, dont la tige 47 est relié au chariot 37 par une tige 48.

Ainsi, l'axe 11 peut coulisser dans une fente 49, de la plaque de base 38, parallèle au rail 45 et orthogonal à la barrette 35 de diodes CCD.

Le chariot 37 peut porter de plus un vibreur 49 destiné à animer épisodiquement, à l'encontre de l'action d'un ressort 50, l'axe 11 d'un mouvement alternatif parallèlement à son axe, afin d'agiter éventuellement le liquide contenu dans les alvéoles 13 du disque 12.

Quand les alvéoles $13_1$ à $13_p$ du rayon 19 se trouvant dans le plan défini par les axes 6 et 11 doivent être lus par l'agencement 35 pour détecter et quantifier les agglutinations (voir la figure 8), le disque 12 est avancé par le vérin 46 dans le sens de la flèche $F_1$ pour que l'agencement 35 soit à l'aplomb de la limite externe 51 du champ 25 du fond du premier alvéole $13_1$. Puis, pas à pas le vérin est déplacé $\underline{m}$ fois pour que les $\underline{n}$ éléments photosensibles de l'agencement 35 examine en n x m points ledits champs 25. Lorsque la lecture du champ 25 de l'alvéole $13_1$ est terminée, le vérin 46 déplace l'axe 11, toujours dans le sens $F_1$, de façon que la limite externe 52 du champ 25 du fond du second alvéole $13_2$ arrive à l'aplomb des éléments photosensibles de l'agencement 35. Ce second alvéole $13_2$ est lu de façon identique et le processus se continue jusqu'à la lecture complète de l'alvéole $13_p$. Après celle-ci, le vérin 46 ramène l'axe 11 dans sa position initiale (flèche $F_2$).

## Revendications

1.- Dispositif pour la détection et la quantification, dans des récipients (13) dont au moins les fonds (25) sont transparents, d'agglutinats susceptibles d'être formés, sous l'action d'au moins un réactif liquide, par des particules en suspension dans des doses de liquides à tester se trouvant dans lesdits récipients, dispositif comportant un agencement linéaire (35) d'une pluralité $\underline{n}$ d'éléments photosensibles observant par transparence les fonds (25) desdits récipients (13) contenant les éventuels agglutinats en balayant lesdits fonds des récipients, de façon à former n x m points d'observation répartis selon une surface rectangulaire ou carrée occupant la plus grande partie de la surface du fond de chaque récipient, lesdits récipients étant constitués d'alvéoles (13) répartis selon des rayons (19) d'un disque (12) horizontal transparent pouvant tourner autour de son axe vertical 11, caractérisé en ce que:
- un bras oscillant (5) tournant autour d'un axe vertical (6) et pourvu de moyens (7) de prélèvement et de restitution de doses liquides, est prévu pour prélever de telles doses dans des réservoirs à réactifs (9) et à liquides à tester (26) et les amener dans lesdits alvéoles (13) du disque transparent (12);
- ledit agencement linéaire d'éléments photosensibles (35) est disposé horizontalement à poste fixe orthogonalement à un diamètre du disque (12); et
- l'axe de rotation (11) du disque (12) est monté mobile en translation orthogonalement audit agencement (35) d'éléments photosensibles.

2.- Dispositif selon la revendication 1, caractérisé en ce que le bras oscillant (5) porte autant de moyens (7) de prélèvement et de restitution qu'un rayon (19) du disque (12) comporte d'alvéoles (13).

3.- Dispositif selon la revendication 1, caractérisé en ce que l'agencement linéaire (35) d'éléments photosensibles est disposé orthogonalement au plan vertical passant par les axes verticaux (11) du disque (12) et (6) du bras oscillant (5), du côté de l'axe (11) du disque (12) opposé audit bras oscillant (5).

4.- Dispositif selon la revendication 1, caractérisé en ce que les réservoirs (9) à réactifs sont disposés à poste fixe d'un côté du plan vertical défini par les axes verticaux (6 et 11) du bras oscillant (5) et du disque (12, tandis que les emplacements de prélèvement (34) des réservoirs (26) à liquides à tester sont disposés de l'autre côté dudit plan vertical.

5.- Dispositif selon la revendication 4, caractérisé en ce que les réservoirs (26) contenant les liquides à tester sont mobiles et amenés à l'emplacement de prélèvement (34) par un transporteur.

6.- Dispositif selon l'une quelconque des revendications 1 à 5, caractérisé en ce que l'agencement (35) d'éléments photosensibles est une barrette de diodes CCD.

7.- Dispositif selon la revendication 1, caractérisé en ce que l'axe (11) du disque (12) est solidaire d'un chariot (37), mobile en coulissement le long d'un rail (45) et portant un moteur (40) pour l'entraînement en rotation dudit axe (11).

8.- Dispositif selon la revendication 7, caractérisé en ce que le chariot (37) est mû par un vérin.

9.- Dispositif selon la revendication 7, caractérisé en ce que l'axe (11) du disque (12) est monté dans un palier lisse solidaire du chariot (37) et en ce qu'un vibreur (49) peut faire vibrer l'axe (11) longitudinalement.

## Patentansprüche

1. Vorrichtung zum Prüfen und quantitativen Bestimmen, in Behältern (13), deren zumindest Böden (25) durchsichtig sind, von unter der Wirkung zumindest eines flüssigen Reagens formbaren Agglutinats durch sich in den Dosen der zu prüfenden Flüssigkeiten in den Behältern in Suspension befindlichen Teilchen, und bestehend aus einer linearen Anordnung (35) mit einer Anzahl $\underline{n}$ photosensibler Elemente, durch die in der Durchsicht durch die Böden (25) der Behälter (13) beobachtet werden, in denen die etwaigen Agglutinate enthalten sind, indem die Behälterböden derart abgetastet oder überstrichen werden, daß n x m Beobachtungspunkte verteilt entlang einer rechtwinkeligen oder quadratischen Oberfläche gebildet werden, die den größten Teil der Fläche des Bodens jedes Behälters einnimmt, wobei die Behälter aus Zellen (13) gebildet sind, die längs der Radien (19) einer horizontalen durchsichtigen Scheibe (12) verteilt angeordnet sind, die um ihre senkrechte Achse (11) drehbar ist, dadurch gekennzeichnet, daß

- ein um eine senkrechte Achse (6) drehbarer Schwenkarm (5) mit einer Einrichtung (7) zum Entnehmen und Rückstellen der Flüssigkeitsdosen vorgesehen ist zum Entnehmen der Dosen aus dem Reagenzien (9) und Prüfflüssigkeiten (26) enthaltendem Vorratsbehälter und zum Einbringen derselben in die Zellen (13) der durchsichtigen Scheibe (12);

- die lineare Anordnung photosensibler Elemente (35) horizontal feststehend orthogonal zu einem Durchmesser der Scheibe (12) angeordnet ist, und daß

- die Drehachse (11) der Scheibe (12) translationsbeweglich orthogonal zur Anordnung (35) der photosensiblen Elemente (35) eingesetzt ist.

2. Vorrichtung nach Anspruch 1, dadurch gekennzeichnet, daß der Schwenkarm (5) ebensoviele Einrichtungen (7) zum Entnehmen und Rückstellen aufweist, wie ein Radius (19) der Scheibe (12) Zellen (13) besitzt.

3. Vorrichtung nach Anspruch 1, dadurch gekennzeichnet, daß die lineare Anordnung (35) photosensibler Elemente orthogonal zur senkrechten Ebene liegt, die durch die senkrechten Achsen (11) der Scheibe (12) und (6) des Schwenkarms (5) auf der Seite der Achse (11) der Scheibe (12) gegenüber vom Schwenkarm (5) verläuft.

4. Vorrichtung nach Anspruch 1, dadurch gekennzeichnet, daß die Vorratsbehälter (9) für Reagenzien feststehend auf der einen Seite der senkrechten Ebene, die durch die senkrechten Achsen (6 und 11) des Schwenkarms (5) und der Scheibe (12) definiert ist, angeordnet sind, während die Standorte der Entnahme (34) der die Prüfflüssigkeiten enthaltenden Vorratsbehälter (26) auf der anderen Seite der senkrechten Ebene liegen.

5. Vorrichtung nach Anspruch 4, dadurch gekennzeichnet, daß die die Prüfflüssigkeiten enthaltenden Vorratsbehälter (26) beweglich sind und von einem Beförderungsmittel der Entnahmestelle (34) zugeführt werden.

6. Vorrichtung nach einem der vorhergehenden Ansprüche 1 bis 5, dadurch gekennzeichnet, daß für die Anordnung (35) der photosensiblen Elemente eine Diodenschiene CCD verwendet wird.

7. Vorrichtung nach Anspruch 1, dadurch gekennzeichnet, daß die Achse (11) der Scheibe (12) mit dem Wagen (37) verbunden ist, der längs der Schiene (45) gleitend verschiebbar ist und einen Motor (40) zum Drehantrieb der Achse (11) trägt.

8. Vorrichtung nach Anspruch 7, dadurch gekennzeichnet, daß der Wagen (37) von einem Stellantrieb verschoben wird.

9. Vorrichtung nach Anspruch 7, dadurch gekennzeichnet, daß die Achse (11) der Scheibe (12) auf einem mit dem Wagen (37) verbundenem Gleitlager montiert ist und daß ein Schwingungserreger (49) die Achse (11) in Längsrichtung schwingen lassen kann.

**Claims**

1.- Device for the detection and quantification, in recipients (13) of which at least the bottoms (25) are transparent, of agglutinates capable of being formed under the action of at least one liquid reagent by particles in suspension in doses of liquids to be tested contained in said recipients, device comprising a linear arrangement (35) of a plurality n of photosensitive elements observing by transparency the bottoms (25) of said recipients (13) containing the possible agglutinates by scanning said bottoms of said recipients, so as to form n x m points of observation distributed over a rectangular or square surface occupying the major part of the surface of the bottom of each recipient, said recipients being constituted by pits (13) distributed along radii (19) of a transparent horizontal disc (12) adapted to rotate about its vertical axis (11), characterized in that:

- an oscillating arm (5) rotating about a vertical axis (6) and provided with means (7) for taking and restituting liquid doses is provided to take such doses from reservoirs of reagents (9) and of liquids to be tested (26) and for bringing them into said pits (13) in the rotating disc (12);

- said linear arrangement (35) of photosensitive elements is permanently disposed horizontally at right angle to a diameter of the disc (12); and

- the axis of rotation (11) of the disc (12) is mounted to move in translation at right angle to said linear arrangement (35) of photosensitive elements.

2.- Device according to claim 1, characterized in that the oscillating arm (5) bears as many taking and restituting means (7) as a radius (19) of the disc (12) comprises pits (13).

3.- Device according to claim 1, characterized in that the linear arrangement (35) of photosensitive elements is disposed at right angle to the vertical plane passing through the vertical axes (11) of the disc (12) and (6) of the oscillating arm (5), on the side of the axis (11) of the disc (12) opposite said oscillating arm (5).

4.- Device according to claim 1, characterized in that the reservoirs (9) of reagents are disposed permanently on one side of the vertical plane defined by the vertical axes (6 and 11) of the oscillating arm (5) and the disc (12), whilst the stations (34) for taking from the reservoirs (26) of liquids to be tested are disposed on the other side of said vertical plane.

5.- Device according to claim 4, characterized in that the reservoirs (26) containing the liquids to be tested are mobile and brought to said stations (34) by a conveyor.

6.- Device according to any one of claims 1 to 5, characterized in that the arrangement (35) of photosensitive elements is a bar of CCD diodes.

7.- Device according to claim 1, characterized in that the spindle (11) of the disc (12) is fast with a carriage (37) slidable along a rail (45) and bearing a motor (40) for driving said spindle (11) in rotation.

8.- Device according to claim 7, characterized in that the carriage (37) is moved by a jack.

9.- Device according to claim 7, characterized in that the spindle (11) of the disc (12) is mounted in a smooth bearing fast with the carriage (37) and a vibrating means (49) may cause the spindle (11) to vibrate longitudinally.

Fig.1

0 107 580

Fig. 2

Fig. 4

Fig. 3

0 107 580

Fig.5

Fig.8

5

Fig:6

Fig. 7